Europäisches Patentamt

European Patent Office

Office européen des brevets

(19)

(11) Veröffentlichungsnummer: **0 302 953**

**A1**

(12) # EUROPÄISCHE PATENTANMELDUNG

(21) Anmeldenummer: 87111588.7

(51) Int. Cl.⁴: **C08L 89/06 , A61L 31/00**

(22) Anmeldetag: **11.08.87**

(43) Veröffentlichungstag der Anmeldung:
**15.02.89 Patentblatt 89/07**

(84) Benannte Vertragsstaaten:
**AT BE CH DE ES FR GB GR IT LI LU NL SE**

(71) Anmelder: **Deutsche Gelatine-Fabriken Stoess & Co.GmbH
Postfach 100 Gammelsbacher Strasse 2
D-6930 Eberbach(DE)**

(72) Erfinder: **Bräumer, Klaus, Dr.
6, Allensteinerstrasse
D-6930 Eberbach(DE)**
Erfinder: **Fischer, Gerhard, Dr.
28/1, Hohenstaufenstrasse
D-6930 Eberbach(DE)**
Erfinder: **Koepff, Peter, Dr.
142, Bergstrasse
D-6900 Heidelberg(DE)**

(74) Vertreter: **Werner, Hans-Karsten, Dr. et al
Deichmannhaus am Hauptbahnhof
D-5000 Köln 1(DE)**

(54) Formkörper aus Kollagen und Kollagenabbauprodukten und Verfahren zur Herstellung derselben.

(57) Formkörper aus Kollagen und Kollagenabbauprodukten, ausgenommen Gelatinekapseln mit gesteuerter Wirkstofffreisetzung, enthalten 0,001 bis 10 Gew.-%, vorzugsweise 0,01 bis 2 Gew.-% physiologisch und toxikologisch unbedenkliche Aldehyde mit mindestens 4 Kohlenstoffatomen. Die Herstellung erfolgt durch Zusatz dieser Aldehyde zur Gelatinelösung, Kollagenlösung bzw. festen Kollagenaufbereitung.

EP 0 302 953 A1

## Formkörper aus Kollagen und Kollagenabbauprodukten und Verfahren zur Herstellung derselben

Gegenstand der vorliegenden Erfindung sind Formkörper aus Kollagen und Kollagenabbauprodukten und Verfahren zur Herstellung derselben, mit Ausnahme von Gelatinekapseln mit gesteuerter Wirkstofffreisetzung.

Formkörper aus Kollagen und Kollagenabbauprodukten werden zu den verschiedensten Zwecken und in den verschiedensten Formen hergestellt. Außer Gelatinekapseln werden schon jetzt die folgenden Formkörper aus Kollagen und Kollagenabbauprodukten hergestellt und verwendet: Folien und Schäume zur Wundabdeckung, in der Chirurgie und Kieferchirurgie als Füllmassen und Tampons sowie Gesichtsmasken in der Kosmetik, Mikrokapseln, Folien zur Verpackung von Lebensmitteln, Wursthüllen und eßbare Wursthüllen und vernetztes lösliches Kollagen zur subkutanen Behandlung von Falten. Auch hierbei ist der gezielte biologische und/oder physiologische Abbau einerseits und eine gezielte Härtung andererseits von entscheidender Bedeutung. Um den Abbau zu verzögern, ist es bekannt, die fertigen Formkörper nachträglich mit Formaldehyd, Glyoxal, Glutardialdehyd oder Hexamethylendiisocyanat zu behandeln und dadurch zu härten. Ein Nachteil dieses Verfahrens besteht darin, daß die Härtung des Kollagens und der Kollagenabbauprodukte nur schwer steuerbar ist, da die Reaktivität des Formaldehyds und der anderen Aldehyde auf die Formkörper aus Kollagen und Kollagenabbauprodukten sehr unterschiedlich sein kann und vor allem stark zeitabhängig ist. In der Zwischenzeit ist Formaldehyd in den Verdacht geraten, kanzerogen zu sein, so daß ein völliges Verbot für die freie Benutzung dieses Stoffes zu befürchten ist. Auch Glyoxal, Glutardialdehyd oder Hexamethylendiisocyanat werden nicht mehr als gesundheitlich unbedenklich angesehen.

Gegenstand der europäischen Patentanmeldung 86 104 655.5 sind Gelatinekapseln mit gesteuerter Wirkstofffreisetzung, gekennzeichnet durch einen Gehalt von 0,001 bis 10 Gew.-%, vorzugsweise 0,01 bis 2 Gew.-% physiologisch und toxikologisch unbedenklicher Aldehyde mit mindestens 4 Kohlenstoffatomen.

Es wurde jetzt gefunden, daß auch andere Formkörper aus Kollagen und Kollagenabbauprodukten mit einem Gehalt von 0,001 bis 10 Gew.-%, vorzugsweise 0,01 bis 2 Gew.-% physiologisch und toxikologisch unbedenklicher Aldehyde mit mindestens 4 Kohlenstoffatomen überraschend einfach hergestellt werden können und überraschend gute Eigenschaften aufweisen. Als Aldehyde sind insbesondere Terpene, Zimtaldehyde, Aldosen oder Gemische derselben geeignet. Besonders bewährt haben sich etherische Öle mit einem hohen Gehalt an Aldehyden wie Citral oder Citral in reiner Form, Zimtaldehyd sowie die Aldosen, Xylose und Arabinose.

Erstaunlicherweise ist es möglich, diese Aldehyde der Gelatinelösung zuzugeben und diese dann in an sich bekannter Weise zu Formkörpern zu verarbeiten, ohne daß es dabei zu Störungen kommt. Von Formaldehyd und Glutardialdehyd ist nämlich bekannt, daß sie bereits bei Zugabe zur Gelatinelösung mit der Gelatine so stark reagieren, daß es zu einem starken Anstieg der Viskosität kommt, der die technische Weiterverarbeitung stört oder sogar unmöglich macht. Überraschenderweise sind die erfindungsgemäß verwendeten Aldehyde mit mindestens 4 Kohlenstoffatomen in der Lage, bereits in relativ geringen Mengen die Eigenschaften der fertigen Formkörper zu verändern, ohne daß es dabei zu Schwierigkeiten bei der Verarbeitung der Gelatinelösung kommt. Dies ist um so erstaunlicher, als die Gelatinelösung auf Temperaturen von mindestens 50° C gebracht und gehalten werden muß, die Trocknung hingegen bei niedrigeren Temperaturen bis hin zur Raumtemperatur erfolgt.

Das erfindungsgemäße Verfahren zur Herstellung von Formkörpern aus Kollagen und Kollagenabbauprodukten, ausgenommen Gelatinekapseln mit gesteuerter Wirkstofffreisetzung, ist somit dadurch gekennzeichnet, daß der Gelatinelösung oder Kollagenpräparation 0,001 bis 10 Gew.-%, vorzugsweise 0,01 bis 2 Gew.-% physiologisch und toxikologisch unbedenklicher Aldehyde mit mindestens 4 Kohlenstoffatomen zugegeben werden und diese Grundmassen in an sich bekannter Weise zu Formkörpern verarbeitet werden.

Durch den Zusatz der erfindungsgemäß verwendeten Aldehyde ist es möglich, je nach Art und Menge die Wiederauflösung der Formkörper mehr oder weniger zu verzögern bzw. ihnen die gewünschte Festigkeit zu geben.

Ein besonderer Vorteil erfindungsgemäß hergestellter Formkörper ist die gute Reproduzierbarkeit der erzielten Ergebnisse, wobei im Gegensatz zur nachträglichen Behandlung der Kollagene und Kollagenabbauprodukte mit Formaldehyd der Zeitfaktor offensichtlich keine Rolle mehr spielt. Dies ist wahrscheinlich darauf zurückzuführen, daß die Aldehyde bereits unmittelbar nach der Zugabe zur Gelatinelösung, Kollagenlösung bzw. festen Kollagenaufbereitung bei den erhöhten Temperaturen mit diesen reagieren, die Vernetzung jedoch erst in der zweiten Phase des Trocknens stattfindet. Da es sich obendrein um physiologisch und toxikologisch unbedenkliche Aldehyde handelt, würden auch nicht umgesetzte Reste oder wieder abgespaltene Reste der Aldehyde unschädlich sein. Da keine organischen Lösungsmittel zur Anwendung kommen, ist das erfindungsgemäße Verfahren bezüglich Arbeitssicherheit und Umweltbelastung besonders vorteilhaft.

Ein weiterer Vorteil der erfindungsgemäßen Formkörper besteht darin, daß sich jetzt auch dort Gelatine und Kollagen als Material problemlos einsetzen lassen, wo bisher weitgehend andere Materialien eingesetzt wurden - beispielsweise in der Hirnchirurgie, wo bisher vielfach Titanfolien eingesetzt wurden, sowie gegebenenfalls auch zur Nagelung gebrochener Knochen.

In den folgenden Beispielen ist die Erfindung näher erläutert.

## Beispiel 1 (Gelatineschaum)

Man versetzt Gelatinepulver mit der notwendigen Menge an kaltem Wasser. Dieses Gemisch läßt man 30 Minuten quellen. Anschließend wird durch Erwärmen auf 60° C die Gelatine in Lösung gebracht. Vor dem Aufschäumen werden der Lösung 0,5% Citral, bezogen auf trockene Gelatine, zugesetzt. Diese Lösung wird nun mit einem sterilen Gas auf das 30-fache des Ausgangsvolumens aufgeschäumt. Der erstarrte Schaum wird in üblicher Weise bis zu einer Restfeuchtigkeit von 9 bis 13%, bezogen auf Trockenstoff, getrocknet. Während unvernetzter Gelatineschaum in Wasser zerfällt, kann der so hergestellte Schaum, getränkt mit wäßrigen Lösungen, manipuliert werden, ohne daß er zerfällt.

Der Vernetzungsgrad kann durch Variation der Citralkonzentration so eingestellt werden, daß er den Bestimmungen über das Resorptionsverhalten Gelatin Sponge der USP XX entspricht.

## Beispiel 2 (Gelatinefolie)

1 kg Gelatine werden in 2 l kaltem Wasser aufgequollen und nach 30 Minuten durch Erwärmen auf 60° C in Lösung gebracht. In diese Lösung werden 3 g Zimtaldehyd eingerührt. Anschließend wird die Lösung mit Hilfe eines Gießrakels auf ein gekühltes Förderband aufgetragen, auf dem der gegossene Gelatinefilm erstarrt. Dieser wird auf ein kunststoffbeschichtetes Förderband übertragen und in der üblichen Weise schonend getrocknet. Im Gegensatz zu unvernetztem Gelatinefilm ist dieser beim Einlegen in Wasser stabil und löst sich auch bei Erwärmen auf 60° C nicht mehr auf.

## Beispiel 3 (Kollagenschaum)

1 kg Korium aus Rinderhaut wird in üblicher Weise durch Behandlung mit einer beliebigen Säure auf pH 3 eingestellt. Das stark gequollene Korium wird nun mit 15 l Wasser versetzt und in üblicher Weise zu einem Brei vermahlen. In diesen Kollagenbrei werden 2 g Arabinose eingemischt. Die Mischung gibt man in Formen und gefriert diese bei -30° C ein. Die gefrorene Kollagenmasse läßt man weitere 24 Stunden bei -8° C stehen. Danach wird diese aufgetaut und das enthaltene Wasser durch herkömmliche Trocknungsverfahren entfernt. Anschließend wird der trockene Schaum für 1 Stunde auf 80° C erhitzt. Der so erhaltene Schaum ist gegenüber wäßrigen Lösungen stabil und manipulierbar und zeigt eine verzögerte physiologische Resorptionszeit.

## Beispiel 4 (eßbare Wursthüllen)

Korium von Rinderhaut wird 22 Tage mit gesättigter Kalklösung behandelt. Anschließend wird der Kalk ausgewaschen und in üblicher Weise der pH-Wert auf pH 3 eingestellt. Die gequollenen Hautstücke werden unter Zusatz von Wasser und gegebenenfalls anderen Zusatzstoffen zu einem Faserbrei mit einem Gehalt von 10% Kollagen vermahlen.

In diesen Brei werden 1,5% Xylose, bezogen auf Kollagen, homogen eingemischt. Anschließend wird in bekannter Weise der Faserbrei durch Ringschlitzdüsen extrudiert und in aufgeblasenem Zustand schonend getrocknet. Man erhält eine Endloswursthülle, die in ihren Eigenschaften den künstlich hergestellten kollagenen Wursthüllen entspricht, die mit Holzrauchdestillat, Formalin, Glyoxal, Glutardialdehyd oder Hitzebehandlung vernetzt werden.

## Ansprüche

1. Formkörper aus Kollagen und Kollagenabbauprodukten, ausgenommen Gelatinekapseln mit gesteuerter Wirkstofffreisetzung, gekennzeichnet durch einen Gehalt von 0,001 bis 10 Gew.-%, vorzugsweise 0,01 bis 2 Gew.-% physiologisch und toxikologisch unbedenklicher Aldehyde mit mindestens 4 Kohlenstoffatomen.

2. Formkörper gemäß Anspruch 1, dadurch gekennzeichnet, daß als Aldehyde Terpene, Zimtaldehyde, Aldosen oder Gemische derselben verwendet werden.

3. Verfahren zur Herstellung von Formkörpern aus Kollagen und Kollagenabbauprodukten, ausgenommen Gelatinekapseln mit gesteuerter Wirkstofffreisetzung, dadurch gekennzeichnet, daß der Gelatinelösung, Kollagenlösung oder festen Kollagenaufbereitung 0,001 bis 10 Gew.-%, vorzugsweise 0,01 bis 2 Gew.-% physiologisch und toxikologisch unbedenklicher Aldehyde mit mindestens 4 Kohlenstoffatomen zugegeben werden und diese Grundmassen in an sich bekannter Weise zu Formkörpern verarbeitet werden.

4. Verfahren gemäß Anspruch 3, dadurch gekennzeichnet, daß als Aldehyde Terpene, Zimtaldehyde, Aldosen oder Gemische derselben verwendet werden.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-B-1 295 989 (JOHNSON & JOHNSON)<br>* Spalte 1, Zeilen 45-60; Ansprüche *<br>----- | 1-4 | C 08 L 89/06<br>A 61 L 31/00 |
| | | | **RECHERCHIERTE SACHGEBIETE (Int. Cl.4)**<br><br>C 08 H<br>C 08 L<br>A 61 K<br>A 22 C<br>A 61 L |

Der vorliegende Recherchenbericht wurde für alle Patentansprüche erstellt

| Recherchenort | Abschlußdatum der Recherche | Prüfer |
|---|---|---|
| DEN HAAG | 15-04-1988 | LENSEN H.W.M. |